# EUROPÄISCHE PATENTANMELDUNG

(11) **EP 4 830 760 A1**
(43) Veröffentlichungstag der Anmeldung: **28.10.2026**
(21) Anmeldenummer: 25172098.3
(22) Anmeldetag: 23.04.2025
(51) Int. Cl.: A61M 60/178, A61M 60/216, A61M 60/531

(54) **STEUEREINHEIT ZUR STEUERUNG EINER HERZPUMPE**

(71) Anmelder: Berlin Heart GmbH, 12247 Berlin (DE); Medizinische Universität Wien, 1090 Wien (AT)
(72) Erfinder: GRANEGGER, Marcus, 2380 Perchtoldsdorf (AT)
(74) Vertreter: Pfenning, Meinig & Partner mbB

(57) **Zusammenfassung**

Diese Offenbarung beschreibt eine Steuereinheit (100) zur Steuerung einer Herzpumpe zur mechanischen Unterstützung eines Herzens Hierzu umfasst die Steuereinheit (100) eine Drucksignalschnittstelle (102), die ausgebildet ist, ein oder mehrere Drucksignale, die einen Druckdifferenzwert (104) entsprechend eines Füllungsdruckunterschieds zwischen einer rechten Herzhälfte und einer linken Herzhälfte des Herzens angeben, oder anhand derer dieser Druckdifferenzwert ermittelbar ist, zu empfangen. Ferner umfasst die Steuereinheit (100) eine Speichereinheit (110), die ausgebildet ist, einen Druckdifferenzsollwert (112) bereitzustellen. Weiterhin umfasst die Steuereinheit (100) eine Recheneinheit (120), die ausgebildet ist, den Druckdifferenzwert (104) und den Druckdifferenzsollwert (112) zu empfangen und in einem Differenzwertbetriebsmodus einen Betriebsparameter (122) der Herzpumpe in Abhängigkeit des Druckdifferenzwertes (104) und des Druckdifferenzsollwertes (112) zu ermitteln. Schließlich umfasst die Steuereinheit eine Steuersignalschnittstelle (132), die ausgebildet ist, ein Steuersignal, das den ermittelten Betriebsparameter (122) angibt, bereitzustellen.

## Beschreibung

Diese Offenbarung betrifft eine Steuereinheit zur Steuerung einer Herzpumpe zur mechanischen Unterstützung eines Herzens, eine Herzpumpenanordnung, die die Steuereinheit umfasst, sowie ein Verfahren zum Betreiben einer Herzpumpe zur mechanischen Unterstützung eines Herzens.

Bei der Realisierung von Herzpumpen werden aufgrund ihrer besonders einfachen Steuerung häufig Rotationspumpen verwendet. Der in dieser Offenbarung beschriebene Ansatz für Herzpumpen zur mechanischen Unterstützung eines Herzen wird daher anhand dieses Pumpentyps illustriert. Andere bezüglich ihrer Förderleistung steuerbaren Pumpentypen sind aber ebenso verwendbar.

Ein einfacher Ansatz zur Steuerung von Rotationsblutpumpen, um das Herz eines Patienten kontinuierlich zu entlasten, ist die Rotationsblutpumpe mit einer konstanten Drehzahl zu betreiben. Dies kann jedoch bei körperlicher Betätigung oder Belastung des Patienten zu einer Minderversorgung im Patientenkörper und/oder zu einer Überlastung des Herzens führen. Die körperliche Leistungsfähigkeit des Patienten ist daher trotz Unterstützungsleistung der Blutpumpe beeinträchtigt.

Um die Beeinträchtigung der körperlichen Leistungsfähigkeit der Patienten zu reduzieren, wurden alternative Ansätze entwickelt. Beispielhaft wird für einen dieser alternativen Ansätze auf die Offenbarung der US 6 623 420 B2 verwiesen. Diese Druckschrift beschreibt eine Blutpumpe, die parallel zwischen dem linken Ventrikel und der Aorta des Herzens eines Patienten angeschlossen ist. Die Pumpe verfügt über einen Einlassdrucksensor und einen kooperierenden Regler zur Messung des Einlassdrucks der Pumpe, um deren Betrieb zu steuern. Der Regler passt die Geschwindigkeit der Pumpe in einem geschlossenen Regelkreis unter Verwendung des gemessenen Einlassdrucks an.

Gegenüber dem Betreiben einer Blutpumpe mit konstanter Drehzahl hat die Steuerung der Blutpumpe entsprechend des Einlassdrucksensors als Approximation des linksventrikulären diastolischen Fülldrucks den Vorteil, dass die Menge des gepumpten Blutes an eine Vorlast des Herzens und somit an die körperliche Betätigung des Patienten angepasst wird.

Allerdings wurde beobachtet, dass bei Patienten, die Herzpumpen, die mit konstanter Drehzahl angesteuert werden, häufiger als normal Rechtherzfehler auftreten. Dies legt nahe, dass eine ideale Regelung einer Blutpumpe nicht nur auf den linksventrikulären Druck optimiert sein sollte, sondern auch den Zustand des rechten Herzens in Betracht ziehen sollte.

Die vorliegende Offenbarung verfolgt daher das Ziel, eine verbesserte Steuerung für eine Blutpumpe zur mechanischen Herzunterstützung zu beschreiben.

Die verbesserte Steuerung wird verwirklicht durch die Steuereinheit zur Steuerung einer Herzpumpe nach Anspruch 1 sowie das Verfahren zum Betreiben einer Herzpumpe nach Anspruch 13. Optionale Merkmale sind in den abhängigen Ansprüchen beschrieben.

Die Steuereinheit zur Steuerung einer Herzpumpe zur mechanischen Unterstützung eines Herzens umfasst eine Drucksignalschnittstelle, eine Speichereinheit, eine Recheneinheit und eine Steuersignalschnittstelle.

Die Drucksignalschnittstelle ist ausgebildet, ein oder mehrere Drucksignale, die einen Druckdifferenzwert entsprechend eines Füllungsdruckunterschieds zwischen einer rechten Herzhälfte und einer linken Herzhälfte des Herzens angeben, oder anhand derer dieser Druckdifferenzwert ermittelbar ist, zu empfangen. Die Speichereinheit ist ausgebildet, einen Druckdifferenzsollwert bereitzustellen. Ferner ist die Recheneinheit ausgebildet, den Druckdifferenzwert und den Druckdifferenzsollwert zu empfangen und in einem Differenzwertbetriebsmodus einen Betriebsparameter der Herzpumpe in Abhängigkeit des Druckdifferenzwertes und des Druckdifferenzsollwertes zu ermitteln. Schließlich ist die Steuersignalschnittstelle ausgebildet, ein Steuersignal, das den ermittelten Betriebsparameter angibt, bereitzustellen.

Der rechte und linke Ventrikel interagieren über das ventrikuläre Septum und beeinflussen sich gegenseitig. Ausgehend von dieser Erkenntnis haben die Erfinder erkannt, dass es für die Steuerung einer Herzpumpe deshalb nicht ausreicht, nur physiologische Parameter derjenigen Seite des Herzens heranzuziehen, die von der Herzpumpe direkt unterstützt wird. Beispielsweise kann im Falle einer linksseitigen Herzunterstützung dies dazu führen, dass die Herzscheidewand (Septum) sich verschiebt und damit die rechte Herzfunktion negativ beeinflusst wird. Durch die Steuerung der Herzpumpe in Abhängigkeit vom Druckunterschied zwischen dem rechten und dem linken Vorhof wird sowohl der Zustand der rechten Herzhälfte als auch der Zustand der linken Herzhälfte berücksichtigt. Hierdurch kann die rechtsventrikuläre Geometrie und die Trikuspidalklappenfunktion erhalten werden ggf. eine rechtventrikuläre Nachlast auf einen Normalwert gesenkt werden und die Förderrate gleichzeitig an den Bedarf der Patienten angepasst werden.

Der Betriebsparameter kann jeglicher Parameter sein, der für eine Steuerung der Herzpumpe verwendet werden kann. Vorzugsweise kann der Betriebsparameter, der von der Recheneinheit ermittelt wird, eine Förderrate der Herzpumpe beeinflussen und/oder diese einstellen. Unter Förderrate wird die Menge an durch die Herzpumpe bewegtem Blut verstanden. Der Betriebsparameter kann beispielsweise eine Drehzahl bzw. Rotationsgeschwindigkeit einer Rotationsblutpumpe, einen Motorstrom, eine Motorspannung und/oder eine Motorleistung der Herzpumpe einstellen.

Ferner wird im Rahmen dieser Offenbarung unter dem Begriff Füllungsdruck derjenige Druck verstanden, der am Ende einer Füllungsphase des Herzens (Diastole) in einem der beiden Herzventrikel (end-diastolischer Druck) herrscht. Der Rechtherzfüllungsdruck gibt dementsprechend den Füllungsdruck im rechten Herzventrikel an. Der Linksherzfüllungsdruck den Füllungsdruck im linken Herzventrikel. Wie später genauer beschrieben wird, kann der Füllungsdruck einer Herzhälfte neben einer Druckermittlung im Ventrikel auch anhand einer Druckermittlung im entsprechenden Vorhof der Herzhälfte ermittelt werden.

Bei der Herzpumpe kann es sich um eine linksventrikuläre Herzpumpe (Left Ventricular Assist Device, LVAD) handeln.

Bei der Speichereinheit kann es sich um einen flüchtigen Speicher, wie beispielsweise um einen Arbeitsspeicher, oder um einen permanenten Speicher, wie beispielsweise eine Festplatte, handeln.

Ferner kann die Recheneinheit ausgebildet sein, den Druckdifferenzwert mit dem Druckdifferenzsollwert zu vergleichen und den Betriebsparameter in Abhängigkeit eines Ergebnisses des Vergleichs zu bestimmen. Die Recheneinheit kann hierfür beispielsweise ausgebildet sein, eine Differenz zwischen Druckdifferenzwert und Druckdifferenzsollwert zu ermitteln. Zudem kann die Recheneinheit ausgebildet sein, den Betriebsparameter in Abhängigkeit des Ergebnisses unter Verwendung eines P-, Pl- oder PID-Regelschemas zu bestimmen.

Weiterhin kann die Recheneinheit ausgebildet sein, zu überprüfen, ob eine oder mehrere Bedingungen, die eine zu hohe Förderrate der Herzpumpe indizieren, erfüllt sind und, in Abhängigkeit eines Ergebnisses dieser Überprüfung, den Druckdifferenzsollwert in der Speichereinheit anzupassen. Zudem kann die Recheneinheit ausgebildet sein, zu überprüfen, ob eine oder mehrere Bedingungen, die eine zu niedrige Förderrate der Herzpumpe indizieren, erfüllt sind und, in Abhängigkeit eines Ergebnisses dieser Überprüfung, den Druckdifferenzsollwert in der Speichereinheit anzupassen. Ausgestaltungen dieser Bedingungen werden im Folgenden beschrieben.

Beispielsweise können die ein oder mehreren Drucksignale einen Rechtsherzfüllungsdruck angeben oder der Rechtsherzfüllungsdruck kann anhand dieser ermittelbar sein. In dieser Ausführungsform kann die Speichereinheit zusätzlich ausgebildet sein, wenigstens einen Rechtsherzfüllungsdruckgrenzwert bereitzustellen. Zudem kann die Recheneinheit ausgebildet sein, den Rechtsherzfüllungsdruck mit dem wenigstens einen Rechtsherzfüllungsdruckgrenzwert zu vergleichen und in Abhängigkeit von einem Ergebnis des Vergleichs, den Druckdifferenzsollwert in der Speichereinheit anzupassen.

In Ausführungsformen, in denen der Druckdifferenzsollwert in Abhängigkeit des Rechtsherzfüllungsdrucks angepasst wird, kann der wenigstens eine Rechtsherzfüllungsdruckgrenzwert einen unteren Rechtsherzfüllungsdruckgrenzwert umfassen und die Recheneinheit ausgebildet sein, wenn der Rechtsherzfüllungsdruck kleiner ist als der untere Rechtsherzfüllungsdruckgrenzwert, den Druckdifferenzsollwert anzupassen. Vorzugsweise kann dabei die Anpassung des Druckdifferenzsollwertes durch die Recheneinheit so erfolgen, dass, wenn die Druckdifferenz als Rechtsherzfüllungsdruck minus Linksherzfüllungsdruck definiert ist, der Druckdifferenzsollwert verringert wird, und, wenn die Druckdifferenz als Linksherzfüllungsdruck minus Rechtsherzfüllungsdruck definiert ist, der Druckdifferenzsollwert erhöht wird. Hierdurch kann eine Förderrate der Herzpumpe reduziert werden, um einem Ansaugereignis vorzubeugen.

Alternativ oder zusätzlich kann der wenigstens eine Rechtsherzfüllungsdruckgrenzwert einen oberen Rechtsherzfüllungsdruckgrenzwert umfassen und die Recheneinheit ausgebildet sein, wenn der Rechtsherzfüllungsdruck größer ist als der obere Rechtsherzfüllungsdruckgrenzwert, den Druckdifferenzsollwert anzupassen. Vorzugsweise kann dabei die Anpassung des Druckdifferenzsollwertes durch die Recheneinheit so erfolgen, dass, wenn die Druckdifferenz als Rechtsherzfüllungsdruck minus Linksherzfüllungsdruck definiert ist, der Druckdifferenzsollwert erhöht wird, und, wenn die Druckdifferenz als Linksherzfüllungsdruck minus Rechtsherzfüllungsdruck definiert ist, der Druckdifferenzsollwert verringert wird. Hierdurch kann eine Förderrate der Herzpumpe erhöht werden, um das von der rechten Herzhälfte gelieferte Blut schneller wegzupumpen.

Weiterhin kann die Recheneinheit ausgebildet sein, nach dem Bereitstellen des Steuersignals festzustellen, ob ein Ansaugereignis vorliegt, und falls das Ansaugergebnis vorliegt, den Druckdifferenzsollwert in der Speichereinheit anzupassen. Vorzugsweise kann dabei die Anpassung des Druckdifferenzsollwertes durch die Recheneinheit so erfolgen, dass, wenn die Druckdifferenz als Rechtsherzfüllungsdruck minus Linksherzfüllungsdruck definiert ist, der Druckdifferenzsollwert verringert wird, und, wenn die Druckdifferenz als Linksherzfüllungsdruck minus Rechtsherzfüllungsdruck definiert ist, der Druckdifferenzsollwert erhöht wird. Hierdurch kann eine Förderrate der Herzpumpe reduziert werden, um weiteren Ansaugereignissen vorzubeugen.

In einer Variante dieser Ausführungsform, bei der ebenfalls das eine oder die mehreren Drucksignale einen Rechtsherzfüllungsdruck anzeigen oder dieser aus den ein oder mehreren Drucksignalen ermittelbar ist, kann zusätzlich zur Bedingung, dass ein Ansaugereignis vorliegt, eine Bedingung an den Rechtsherzfüllungsdruck, vorzugsweise dass der Rechtsherzfüllungsdruck kleiner ist als ein vorgegebener erster Grenzwert, für die Anpassung des Druckdifferenzsollwertes hinzutreten. In diesem Fall wird der Druckdifferenzsollwert nur angepasst, wenn das Ansaugereignis vorliegt und die Bedingung an den Rechtsherzfüllungsdruck erfüllt ist. Liegt nur ein Ansaugereignis vor und die zusätzliche Bedingung ist nicht erfüllt, kann ein Wechsel in einen anderen, vom Differenzwertbetriebsmodus verschiedenen Betriebsmodus erfolgen.

Weiterhin kann die Recheneinheit in Ausführungsformen der Steuereinheit ausgebildet sein, nach Ermitteln des Betriebsparameters festzustellen, ob der Betriebsparameter außerhalb eines vorgegebenen Betriebsparametersollbereichs liegt, und falls der Betriebsparameter außerhalb des Betriebsparametersollbereichs liegt, den Druckdifferenzsollwert in der Speichereinheit anzupassen. Vorzugsweise kann dabei die Anpassung des Druckdifferenzsollwertes durch die Recheneinheit so erfolgen, dass, wenn die Druckdifferenz als Rechtsherzfüllungsdruck minus Linksherzfüllungsdruck definiert ist, der Druckdifferenzsollwert erhöht wird, und, wenn die Druckdifferenz als Linksherzfüllungsdruck minus Rechtsherzfüllungsdruck definiert ist, der Druckdifferenzsollwert verringert wird. Hierdurch kann beispielsweise verhindert werden, dass der Betriebsparameter einen zu geringen wert annimmt.

Ferner kann die Recheneinheit ausgebildet sein, zu überwachen, ob ein Ansaugereignis vorliegt und gleichzeitig der Betriebsparameter außerhalb eines Betriebsparametersollbereichs liegt, oder ob das Ansaugereignis und der Betriebsparameter außerhalb der Betriebsparametersollbereichs alternierend auftreten, und in Abhängigkeit von einem Ergebnis des Überwachens, in einen anderen, vom Differenzwertbetriebsmodus verschiedenen Betriebsmodus zu wechseln. Zum Überprüfen, ob ein alternierendes Auftreten vorliegt, kann beispielsweise anhand eines vorgegebenen Zeitintervalls und einer vorgegebenen maximalen Anzahl von Wechseln zwischen Ansaugereignis und Betriebsparameter außerhalb des Betriebsparametersollbereichs erfolgen.

In einer Variante dieser Ausführungsform kann die Recheneinheit zusätzlich ausgebildet sein, ein Ansaugloslassverfahren auszuführen, bevor sie in den anderen Betriebsmodus wechselt, und nur dann, wenn das Ansaugloslassverfahren nicht erfolgreich ist, in den anderen Betriebsmodus zu wechseln.

In Ausführungsformen bei der das eine oder die mehreren Drucksignale einen Rechtsherzfüllungsdruck anzeigen oder dieser aus den ein oder mehreren Drucksignalen ermittelbar ist, kann eine zusätzliche Bedingung an den Rechtsherzfüllungsdruck, vorzugsweise dass der Rechtsherzfüllungsdruck größer als ein vorgegebener zweiter Grenzwert ist, für das Ausführen des Ansaugloslassverfahren gegeben sein. Wenn diese Bedingung nicht erfüllt ist, wird der Betriebsmodus ohne das Ausführen des Ansaugloslassverfahren gewechselt.

Weiterhin kann die Recheneinheit ausgebildet sein, vorzugsweise vor dem Bestimmen des Betriebsparameters im Differenzwertbetriebsmodus, zu überprüfen, ob der Druckdifferenzsollwert innerhalb eines vorgegebenen Druckdifferenzsollwertbereichs liegt, und falls der Druckdifferenzsollwert außerhalb des Druckdifferenzsollbereichs liegt, in einen anderen, vom Differenzwertbetriebsmodus verschiedenen Betriebsmodus zu wechseln.

In Ausführungsformen, in denen in einen anderen Betriebsmodus als den Differenzwertbetriebsmodus gewechselt wird, kann der andere Betriebsmodus ein Konstantwertbetriebsmodus sein und die Recheneinheit ausgebildet sein, in dem Konstantwertbetriebsmodus den Betriebsparameter auf einen vordefinierten konstanten Betriebsparameterwert zu setzen.

Ausführungsformen der Steuereinheit können auch umfasst sein von einer Herzpumpenanordnung, die neben der Steuereinheit selbst, einen oder mehrere Drucksensoren und/oder die Herzpumpe umfassen kann. Die Drucksensoren können dabei ausgebildet sein, die ein oder mehreren Drucksignale an der Drucksignalschnittstelle der Steuereinheit bereitzustellen. Ferner kann die Herzpumpe ausgebildet sein, an der Steuersignalschnittstelle bereitgestellte Steuersignale zu empfangen.

Als Drucksensoren können Differenzdrucksensoren oder Absolutdrucksensoren verwendet werden. Beispielsweise wäre es möglich, dass die ein oder mehreren Drucksensoren einen Differenzdrucksensor oder zwei Absolutdrucksensoren oder ein Differenzdrucksensor und einen Absolutdrucksensor umfassen. Bei Verwendung eines Absolutdrucksensors kann ferner eine Korrektur des gemessenen Absolutdrucks unter Berücksichtigung eines ermittelten Atmosphärendrucks durch die Steuereinheit vorgesehen sein. Dazu kann die Steuereinheit eine zusätzliche Korrektureinrichtung umfassen, die beispielsweise mithilfe eines zusätzlichen Drucksensors, der außerhalb des Herzens angeordnet ist, Druckschwankungen außerhalb des Patientenkörpers, die sich auf die Messung des Drucks im Herzen auswirken würden, zu eliminieren.

Die ein oder mehreren Drucksensoren können ausgebildet sein, die ein oder mehreren Drucksignale mittels eines Kabels an der Drucksignalschnittstelle der Steuereinheit bereitzustellen.

Ferner kann die Drucksignalschnittstelle der Steuereinheit für einen kabellosen Empfang von Drucksignalen ausgebildet sein. Um diese Funktion zu nutzen, können die ein oder mehreren Drucksensoren ausgebildet sein, die ein oder mehreren Drucksignale kabellos an der Drucksignalschnittstelle der Steuereinheit bereitzustellen. Ferner können in dieser Ausführungsform die Drucksensoren jeweils einen elektrischen Energiespeicher wie beispielsweise eine Primär- oder Sekundärbatterie zur Bereitstellung der von elektrischer Energie für einen Betrieb des jeweiligen Drucksensors umfassen.

Die Absolutdrucksensoren können für eine Anordnung im linken Vorhof oder Ventrikel und/oder im rechten Vorhof oder Ventrikel des Herzens ausgebildet sein. Der Differenzdrucksensor kann ausgebildet sein für eine Anordnung am Septum des Herzens, vorzugsweise im Bereich des Septums der linken und rechten Vorhof oder im Bereich des Septums der linken oder rechten Ventrikel trennt. Vorzugsweise ist der Differenzdrucksensor so ausgebildet, dass bei einer Anordnung am oder im Septum zum Bestimmen der Druckdifferenz zwischen den Vorhöfen oder der Ventrikel ein Teil des Differenzdrucksensors im rechten Vorhof bzw. Ventrikel und ein anderer Teil des Differenzdrucksensors im linken Vorhof bzw. Ventrikel angeordnet ist.

Die Steuereinheit kann weiterhin eine Eingabeschnittstelle umfassen, die ausgebildet ist, einen Eingabewert zu empfangen. Ferner kann die Recheneinheit ausgebildet sein, in einem Initialisierungsmodus einen Startwert für den Druckdifferenzsollwert zu ermitteln und in der Speichereinheit zu hinterlegen.

In einer Variante hiervon kann es sich bei dem Eingabewert direkt um den zu hinterlegenden Startwert für den Druckdifferenzsollwert handeln. In einer anderen Variante kann es sich bei dem Eingabewert um einen Betriebsparameterwert zum Einstellen einer Förderrate der Herzpumpe handeln. In dieser Variante kann die Recheneinheit zusätzlich ausgebildet sein, das Steuersignal entsprechend dem empfangenen Betriebsparameterwert bereitzustellen und den sich aufgrund des Betriebsparameterwertes einstellenden Füllungsdruck zu ermitteln und als Startwert zu verwenden.

Ferner kann die Steuereinheit eine Ausgabeschnittstelle umfassen und die

Speichereinheit ausgebildet sein, den in der Speichereinheit hinterlegten Druckdifferenzsollwert an der Ausgabeschnittstelle bereitzustellen.

Neben der Steuereinheit wird auch ein Verfahren zum Betreiben einer Herzpumpe zur mechanischen Unterstützung eines Herzens beschrieben. Dieses Verfahren umfasst die folgenden Verfahrensschritte:
- Empfangen eines oder mehrerer Drucksignale, die einen Druckdifferenzwert entsprechend eines Füllungsdruckunterschieds zwischen einer rechten Herzhälfte und einer linken Herzhälfte des Herzens angeben oder anhand derer diese Druckdifferenz ermittelbar ist,
- Bereitstellen eines Druckdifferenzsollwertes,
- in einem Differenzwertbetriebsmodus Ermitteln eines Betriebsparameters der Herzpumpe in Abhängigkeit des Druckdifferenzwertes und des Druckdifferenzsollwertes, und
- Bereitstellen eines Steuersignals, das den ermittelten Betriebsparameter angibt.

Im Folgenden werden Ausführungsbeispiele der Steuereinheit zur Steuerung einer Herzpumpe zur mechanischen Unterstützung eines Herzens, der Herzpumpenanordnung sowie des Verfahrens zum Betreiben einer Herzpumpe zur mechanischen Unterstützung eines Herzens anhand der beiliegenden Figuren beschrieben.

### Dabei zeigen die Figuren:

- Fig. 1: eine Schemazeichnung einer Steuereinheit zur Steuerung einer Herzpumpe zur mechanischen Unterstützung eines Herzens;
- Fig. 2: die Steuereinheit aus Fig. 1 als Teil einer Herzpumpenanordnung;
- Fig. 3: eine Schemazeichnung eines Verfahrens zum Betreiben einer Herzpumpe wie es durch die Steuereinrichtung aus Fig. 1 umgesetzt ist;
- Fig. 4: eine Schemazeichnung einer Steuereinheit zur Steuerung einer Herzpumpe zur mechanischen Unterstützung eines Herzens, die ausgebildet ist einen Druckdifferenzsollwert während des Betriebs anzupassen;
- Fig. 5: ein Regelungsschema zur Illustration von Operationen, die zur Steuerung der Herzpumpe durch eine Recheneinheit der in Fig. 4 dargestellten Steuereinheit durchgeführt werden;
- Fig. 6: eine Schemazeichnung einer Steuereinheit 400, die bei der Steuerung der Herzpumpe den Druck im rechten Vorhof berücksichtigt; und
- Fig. 7: ein Regelungsschema zur Illustration von Operationen, die zur Steuerung der Herzpumpe durch eine Recheneinheit der in Fig. 6 dargestellten Steuereinheit durchgeführt werden.

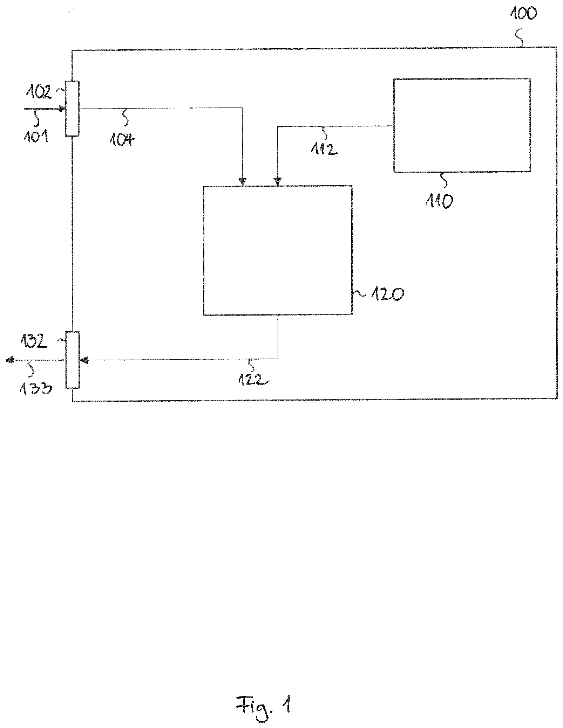

Ein erstes Ausführungsbeispiel einer Steuereinheit zur Steuerung einer Herzpumpe wird im Folgenden anhand von Fig. 1 beschrieben.

Fig. 1 zeigt eine Schemazeichnung einer Steuereinheit 100 zur Steuerung einer Herzpumpe zur mechanischen Unterstützung eines Herzens.

Die Steuereinheit 100 umfasst eine Drucksignalschnittstelle 102, die ausgebildet ist, ein Drucksignal 101, das einen Druckdifferenzwert 104 entsprechend eines Füllungsdruckunterschieds zwischen einer rechten Herzhälfte und einer linken Herzhälfte des Herzens angibt, zu empfangen. Vorzugsweise entspricht der Druckdifferenzwert 104 dem Linksherzfüllungsdruck minus dem Rechtsherzfüllungsdruck.

Ferner umfasst die Steuereinheit 100 eine Speichereinheit 110, die ausgebildet ist, einen Druckdifferenzsollwert 112 bereitzustellen. Der Druckdifferenzsollwert gibt dabei einen Zielwert für die Füllungsdruckdifferenz zwischen linker und rechter Herzhälfte des Herzens des Patienten an, der durch Einsatz der Herzpumpe erreicht werden soll.

Weiterhin umfasst die Steuereinheit 100 eine Recheneinheit 120. Die Recheneinheit 120 ist ausgebildet, den Druckdifferenzwert 104 und den Druckdifferenzsollwert 112 zu empfangen und in einem Differenzwertbetriebsmodus einen Betriebsparameter 122 der Herzpumpe in Abhängigkeit des Druckdifferenzwertes 104 und des Druckdifferenzsollwertes 112 zu ermitteln.

Zudem umfasst die Steuereinheit 100 zur Weitergabe des ermittelten Betriebsparameters 122 an die Herzpumpe eine Steuersignalschnittstelle 132, die ausgebildet ist, ein Steuersignal 133, das den ermittelten Betriebsparameter 122 angibt, bereitzustellen.

Die beschriebene Steuereinheit 100 verwirklicht die Erkenntnis, dass es für die Steuerung einer Herzpumpe nicht ausreicht nur physiologische Parameter derjenigen Seite des Herzens heranzuziehen, die von der Herzpumpe direkt unterstützt wird. Im Falle einer linksseitigen Herzunterstützung kann dies dazu führen, dass die Herzscheidewand (Septum) sich verschiebt und damit die rechte Herzfunktion negativ beeinflusst wird. Durch die Steuerung der Herzpumpe in Abhängigkeit vom Füllungsdruckunterschied zwischen linker und rechter Herzhälfte wird sowohl der Zustand der rechten Herzhälfte als auch der Zustand der linken Herzhälfte berücksichtigt. Hierdurch kann die rechtsventrikuläre Geometrie und die Trikuspidalklappenfunktion erhalten werden, ggf. eine rechtventrikuläre Nachlast auf einen Normalwert gesenkt werden und die Förderrate gleichzeitig an den Bedarf des Patienten angepasst werden.

Die Recheneinheit 120 kann durch eine anwendungsspezifische integrierte Schaltung (engl. ASIC) ausgebildet sein, bei der die Steuerstrategie zumindest teilweise durch die Hardware vorgegeben ist. Alternativ kann die Recheneinheit 120 auch mittels eines Mikrokontrollers oder eines Prozessors realisiert werden, bei dem die Steuerungsstrategie durch ein Computerprogramm vorgegeben ist. Bei der Speichereinheit 110 kann es sich um einen flüchtigen Speicher, wie beispielsweise einem Arbeitsspeicher, oder einen nicht flüchtigen Speicher, wie beispielsweise einer Festplatte, handeln. Die Speichereinheit 110 kann auch Teil eines Prozessors, Mikrokontrollers oder ASICs sein, mittels dem die Recheneinheit 120 realisiert ist. Die Steuereinheit 100 kann sowohl als implantierbare Steuereinheit ausgebildet sein als auch um außerhalb des Körpers des Patienten getragen zu werden.

Die vorgeschlagene Steuerungsstrategie hat das Ziel, die (weniger beeinträchtigte) Funktionalität der Mechanismen der rechtsventrikulären Vorlast und des Herzzeitvolumens auf den versagenden linken Ventrikel zu "spiegeln". Dies kann durch das Ausbalancieren der Füllungsdrücke in der rechten und der linken Herzhälfte erreicht werden. Folglich bestimmt bei dem vorgeschlagenen Steuerungsvorgehen die venöse Physiologie zusammen mit dem rechten Herzen die Betriebsbedingungen des kardiovaskulären Systems. Bei einem linksventrikulären Unterstützungssystem (LVAD) wird so viel Blut gepumpt, wie vom rechten Herzen geliefert wird, und der Linksherzfüllungsdruck an den Rechtsherzfüllungsdruck angepasst, um eine bestimmte Druckdifferenz zwischen Füllungsdrücken zu erreichen. Auf diese Weise wird eine Balance des Vorhof- und Ventrikelseptums während der Diastole sichergestellt.

Um den Betriebsparameter 122 der Herzpumpe in Abhängigkeit vom Druckdifferenzwert 104 und des Druckdifferenzsollwert 112 einzustellen, gibt es verschiedene Regelschemata. Bei der in Fig. 1 gezeigten Ausführungsform ist die Recheneinheit 120 ausgebildet, den Druckdifferenzwert 104 mit dem Druckdifferenzsollwert 112 zu vergleichen und den Betriebsparameter in Abhängigkeit eines Ergebnisses des Vergleichs zu bestimmen. Durch den Vergleich kann eine Abweichung der Werte voneinander bestimmt werden und in Abhängigkeit von der Abweichung, oft auch als Regeldifferenz bezeichnet, der Betriebsparameter 122.

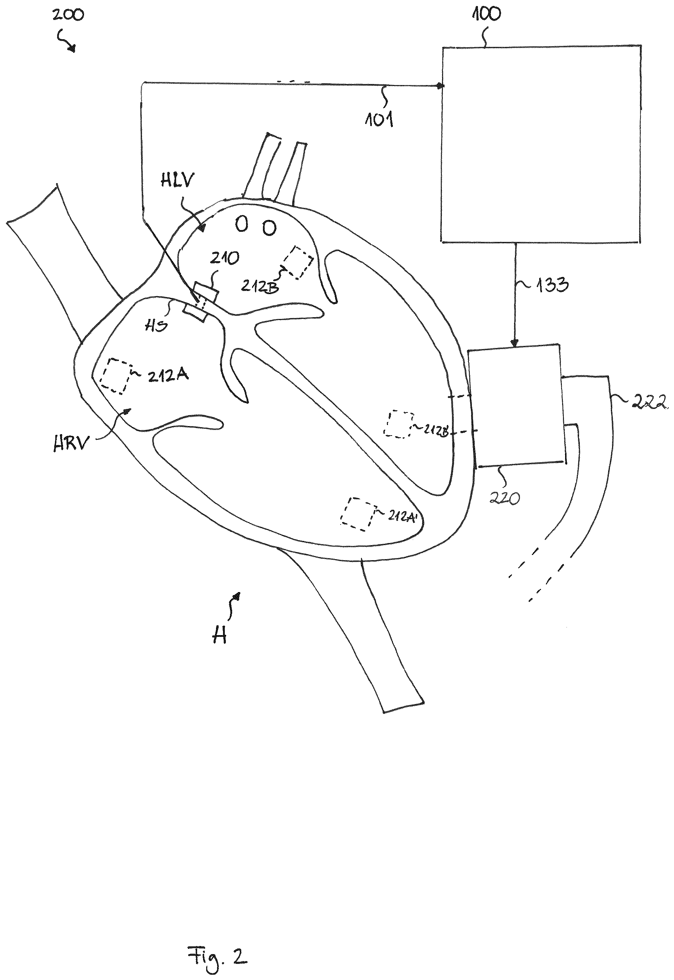

Fig. 2 zeigt die Steuereinheit 100 aus Fig. 1 als Teil einer Herzpumpenanordnung 200.

Die Herzpumpenanordnung 200 ist dargestellt im Kontext eines in Querschnittsansicht gezeigten Herzens H. In der Querschnittsansicht ist ein rechter Vorhof HRV und eine rechte Herzkammer HRK sowie ein linker Vorhof HLV und eine linke Herzkammer HLK des Herzens H dargestellt. Linke und rechte Herzhälfte sind durch Septum HS getrennt.

Die in Fig. 2 dargestellte Herzpumpenanordnung 200 umfasst neben der Steuereinheit 100 eine Herzpumpe 220. Bei der Herzpumpe 220 handelt es sich um eine Linksherzunterstützungssystem (LVAD), das eingerichtet ist, Blut aus der linken Herzkammer HLK zu entnehmen und über eine Fluidverbindung 222 in eine Aorta des Herzens H abzugeben. Hierzu umfasst die Herzpumpe 220 einen Impeller. Ferner ist die Herzpumpe 220 ausgebildet, ein an der Steuersignalschnittstelle 132 der Steuereinheit 100 bereitgestellte Steuersignale 133, das den Betriebsparameter 122 angibt, zu empfangen. Im in Fig. 1 gezeigten Ausführungsbeispiel handelt es sich bei dem Betriebsparameter 122, der durch die Steuereinheit 100 eingestellt wird, um eine Drehzahl des Impellers der Herzpumpe 220. In anderen Ausführungsbeispielen kann die Drehzahl aber auch jeder andere Betriebsparameter sein, mittels dessen sich eine Förderrate der Herzpumpe 220 einstellen bzw. anpassen lässt.

Weiterhin umfasst die Herzpumpenanordnung 200 einen Differenzdrucksensor 210, der beidseitig vom Septum HS im Bereich der Vorhöfe angeordnet ist und ausgebildet ist, in Form eines Drucksignals 101 einen Druckdifferenzwert entsprechend eines Druckunterschieds zwischen dem rechten Vorhof HRV und dem linken Vorhof HLV des Herzens H an der Drucksignalschnittstelle 102 der Steuereinheit 100 bereitzustellen. Durch die Anordnung im Vorhof ist eine indirekte Messung des Füllungsdruckunterschieds zwischen dem linken und rechten Ventrikel möglich. Der Füllungsdruckunterschied zwischen den Herzhälften wird dabei approximiert durch den mittleren gemessenen Druckunterschied zwischen den Vorhöfen über eine Herzperiode. Der Druckdifferenzsensor 210 kann alternativ beidseitig vom Ventrikelseptum angeordnet sein. In diesem Fall entspricht der Füllungsdruckunterschied dem am Ende der Diastole gemessenen Druckdifferenzwert.

Die Datenübertragung zwischen Sensoren und Steuereinheit 100 kann kabelgebunden erfolgen, wie in Fig. 2 angedeutet. Alternativ können die Drucksignalschnittstelle 102 der Steuereinheit 100 wie auch eine entsprechende Schnittstelle des Differenzdrucksensors 210 ausgebildet sein, ein kabellos übertragenes Drucksignal auszutauschen. Hierzu können beispielsweise Technologien wie Near-Field-Communication (NFC), Bluetooth, WLAN oder Zigbee verwendet werden. Ist die Steuereinheit 100 ausgebildet, um außerhalb des Körpers des Patienten getragen zu werden, verläuft eine kabelgebundene Datenübertragung durch eine Durchtrittsstelle in der Haut des Patienten.

Alternativ oder zusätzlich zu dem Differenzdrucksensor 210 kann die Herzpumpenanordnung 200 auch einen Drucksensor 212A und einen Drucksensor 212B umfassen (dargestellt in Fig. 2 mittels gestrichelten Linien). In dem Beispiels von Fig. 2 sind die Drucksensoren 212A,B in den Vorhöfen angeordnet und ausgebildet, den Druck im rechten Vorhof HRV bzw. im linken Vorhof HLV zu messen. Alternativ hierzu können die Drucksensoren 212 A,B auch in den Ventrikeln angeordnet sein, wie durch die mit gestrichelten Linien dargestellten Drucksensoren 212A' und 212B' in Fig. 2 gezeigt ist. Die Ermittlung des Füllungsdruckunterschieds kann dann analog wie beim Differenzdrucksensor 210 ermittelt werden, wobei zunächst der Füllungsdruck für die jeweilige Herzhälfte ermittelt wird und dann anschließend der Differenzwert gebildet wird.

Die für den Füllungsdruckunterschied nötige Drucksignalverarbeitung kann dabei entweder durch ein zur Steuereinheit 100 externes gerät wie beispielsweise den Drucksensoren selbst erfolgen oder in alternativen Ausführungsformen durch die Steuereinheit 100 selbst erfolgen. Im letzten Fall kann diese Aufgabe beispielsweise durch die Recheneinheit 120 übernommen werden.

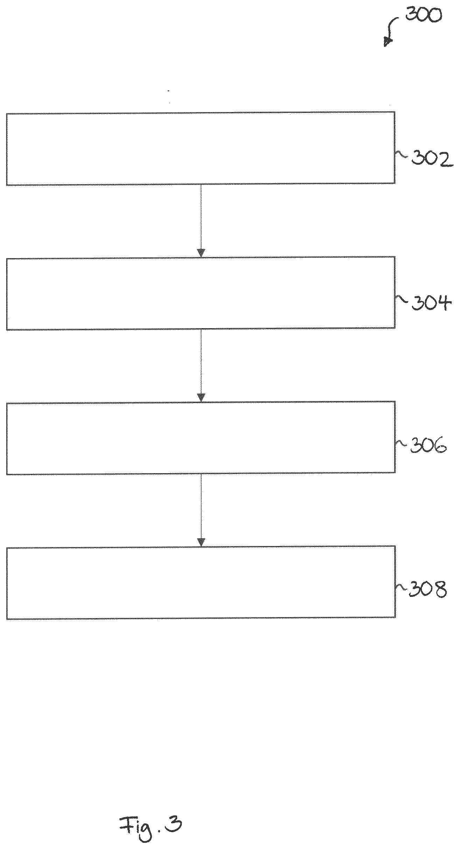

Fig. 3 zeigt eine Schemazeichnung eines Verfahrens 300 zum Betreiben einer Herzpumpe wie es durch die Steuereinrichtung 100 aus Fig. 1 umgesetzt ist. Das Verfahren 300 umfasst insgesamt vier Schritte.

In einem ersten Schritt 302 werden ein oder mehrere Drucksignale empfangen, die einen Druckdifferenzwert entsprechend eines Füllungsdruckunterschieds zwischen der rechten Herzhälfte und der linken Herzhälfte des Herzens angeben oder anhand derer dieser Druckdifferenzwert ermittelbar ist.

In einem zweiten Schritt 304 wird ein Druckdifferenzsollwert bereitgestellt. Schritt 302 und 304 sind in Fig. 3 als zeitlich aufeinanderfolgende Schritte dargestellt. Sie können aber auch in umgekehrter Reinfolge oder zeitlich parallel ausgeführt werden.

In einem dritten Schritt 306 wird in einem Differenzwertbetriebsmodus ein Betriebsparameter der Herzpumpe in Abhängigkeit des Druckdifferenzwertes und des Druckdifferenzsollwertes ermittelt.

In einem vierten Schritt 308 wird ein Steuersignal, das den ermittelten Betriebsparameter angibt, bereitgestellt.

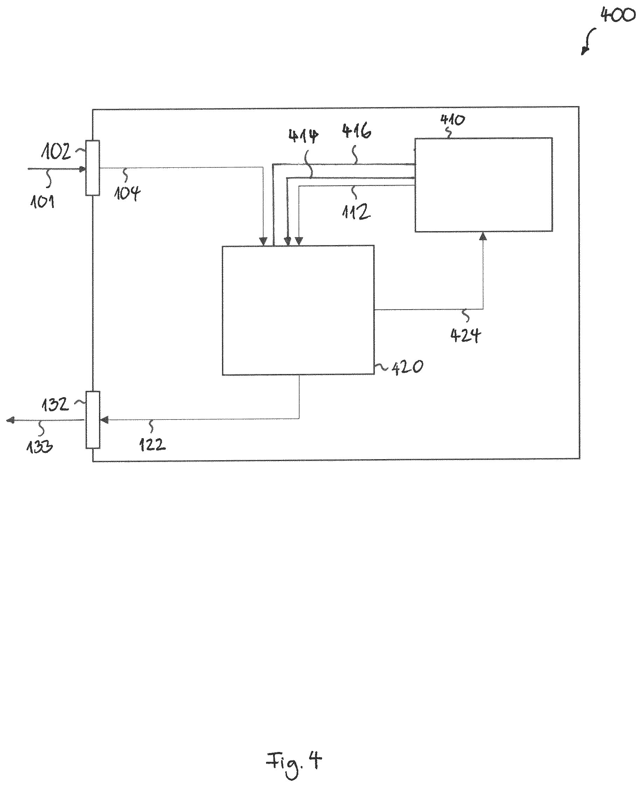

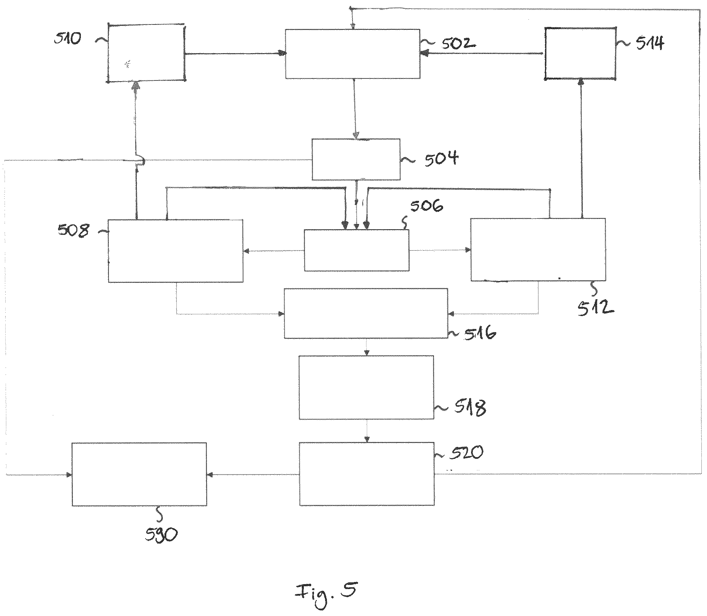

Die Steuereinheit 100 verwendet einen fest vorgegebenen Druckdifferenzsollwert 112 für die Steuerung der Herzpumpe 220. Ändert sich jedoch während des Betriebs die Verfassung des rechten Herzens, kann es sinnvoll sein, den Druckdifferenzsollwert 112 entsprechend anzupassen. Eine Ausführungsform der Steuereinheit, die solch eine Anpassung ermöglicht wird im Folgenden anhand der Fign. 4 und 5 beschrieben.

Fig. 4 zeigt eine Schemazeichnung einer Steuereinheit 400 zur Steuerung einer Herzpumpe zur mechanischen Unterstützung eines Herzens, die ausgebildet ist einen Druckdifferenzsollwert während des Betriebs anzupassen.

Die Steuereinheit 400 ist weitgehend identisch zu der Steuereinheit 100 aus Fig. 1 und unterscheidet sich von dieser nur in Speichereinheit 410 und Recheneinheit 420.

Die Speichereinheit 410 ist ausgebildet neben dem Druckdifferenzsollwert 112 auch einen Druckdifferenzsollwertbereich 414 und einen Betriebsparametersollbereich 416 bereitzustellen.

Die Recheneinheit 420 ist wie die Recheneinheit 120 ausgebildet, den Druckdifferenzwert 104 und den Druckdifferenzsollwert 112 zu empfangen und in einem Differenzwertbetriebsmodus den Betriebsparameter 122 der Herzpumpe in Abhängigkeit des Druckdifferenzwertes 104 und des Druckdifferenzsollwertes 112 zu ermitteln. Zusätzlich ist die Recheneinheit 420 auch ausgebildet, im Druckdifferenzmodus über ein Schreibsignal 424 den in der Speichereinheit 110 gespeicherten Differenzdrucksollwert 112 anzupassen und dabei auch den Druckdifferenzsollwertbereich 414 und den Betriebsparametersollbereich 416 zu berücksichtigen. Wie dies im Detail erfolgt, ist im in Fig. 5 dargestellten Regelungsschema dargestellt.

Fig. 5 zeigt ein Regelungsschema zur Illustration von Operationen, die zur Steuerung der Herzpumpe durch die Recheneinheit 420 der in Fig. 4 dargestellten Steuereinheit 400 durchgeführt werden.

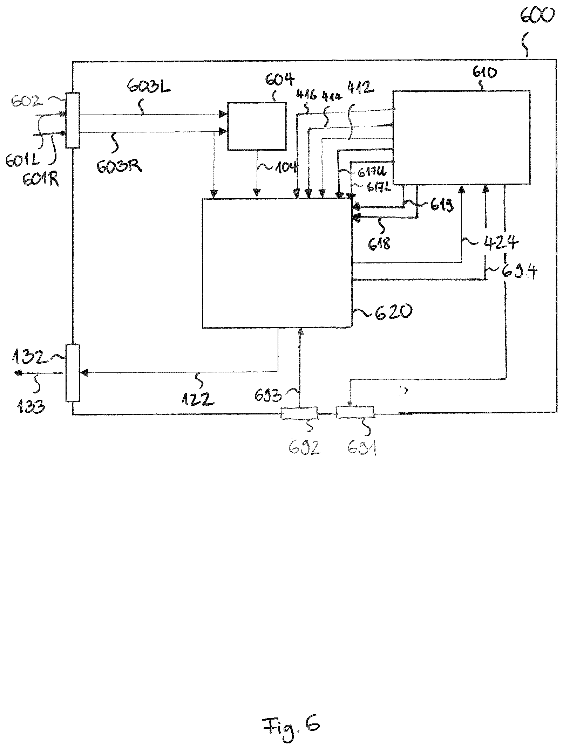

Das Regelungsschema beginnt mit Operation 502, durch die der in der Speichereinheit 110 gespeicherten Druckdifferenzsollwertes 112 zur Verarbeitung abgerufen wird. Der Druckdifferenzwert der initial für das Regelungsschema verwendet wird, kann als konstanter Wert in der Speichereinheit 110 vorgegeben sein. Alternativ kann die Speichereinheit 110 eine Wertetabelle umfassen, in der verschiedene Werte vorgegeben sind, aus denen der Druckdifferenzsollwert entsprechend einem Anfangszustand, beispielsweise einem physiologischen Zustand des Patienten, ausgewählt werden kann. Weitere Möglichkeiten den initial für den Differenzdrucksollwert verwendeten Wert festzulegen, werden im Zusammenhang mit der Beschreibung von Fig. 6 gegeben.

Da der Druckdifferenzsollwert 112 im Druckdifferenzmodus nun veränderlich ist, wird in einer Operation 504 geprüft, ob der Druckdifferenzsollwert in einem vorgegebenen Wertebereich liegt. Hierzu ist die Recheneinheit 420 ausgebildet zu überprüfen, ob der Druckdifferenzsollwert 112 innerhalb des durch die Speichereinheit 410 bereitgestellten Druckdifferenzsollwertbereichs 414 liegt. Beispielsweise könnte der Druckdifferenzsollwertbereich zwischen -5 und +10mmHg liegen. Sofern diese Bedingung erfüllt ist, ist die Recheneinheit 420 ausgebildet, im Differenzwertbetriebsmodus zu verbleiben und in Operation 506 den Betriebsparameter 122 wie zuvor in Bezug auf die Steuereinheit 100 beschrieben in Abhängigkeit des Druckdifferenzsollwertes 112 und des Druckdifferenzwertes 104 zu ermitteln und ein entsprechendes Steuersignal bereitzustellen.

Liegt der Druckdifferenzsollwert jedoch außerhalb des Druckdifferenzsollbereichs 414, ist die Recheneinheit ausgebildet, den Betriebsparameter 122 in einem anderen, vom Differenzwertbetriebsmodus verschiedenen Betriebsmodus zu ermitteln. Dies ist im Regelungsschema der Fig. 5 durch Operation 590 angedeutet. Im Ausführungsbeispiel der Fig. 4 und 5 handelt es sich bei dem anderen, vom Differenzwertbetriebsmodus verschiedenen Betriebsmodus um einen Konstantwertbetriebsmodus. Zur Durchführung der Operation 590 ist die Recheneinheit 420 daher ausgebildet, den Betriebsparameter im Konstantwertbetriebsmodus auf einen vordefinierten konstanten Betriebsparameterwert zu setzen.

Um zu verhindern, dass eine zu hohe Förderrate eingestellt wird, beispielsweise wenn die rechte Herzseite eine Schwächung erfährt, umfasst das Regelungsschema der Fig. 5 eine Operation 508. Zur Durchführung der Operation 508 ist die Recheneinheit 420 ausgebildet, nach dem Bereitstellen des Steuersignals festzustellen, ob ein Ansaugereignis vorliegt. Das Ansaugereignis kann beispielsweise über die in US 2022/152376 A1 oder US 2022/152376 A1 beschriebenen Verfahren erfolgen.

Liegt kein Ansaugereignis vor, kann der Betriebsparameter mit unverändertem Druckdifferenzsollwert 112 bestimmt werden. Dies ist im Regelungsschema durch einen Übergang von Operation 508 zurück zu Operation 506 angedeutet. Falls das Ansaugergebnis jedoch vorliegt, ist eine Anpassung des Druckdifferenzsollwerts 112 nötig. Hierzu ist die Recheneinheit 420 ausgebildet, den Druckdifferenzsollwert 112 in der Speichereinheit 110 anzupassen. Die Anpassung des Druckdifferenzsollwertes 112 erfolgt dabei so, dass die Förderrate der Herzpumpe reduziert wird. Dies kann beispielsweise durch ein Erhöhen des Druckdifferenzsollwertes 112 erreicht werden, wenn die Druckdifferenz als Linksherzfüllungsdruck minus Rechtsherzfüllungsdruck definiert ist. Im Anschluss erfolgt durch nochmaliges Ausführen der Operation 502 eine Fortführung des Regelungsschema mit dem veränderten Druckdifferenzsollwert 112.

Durch die Anpassung des Druckdifferenzsollwertes 112 kann es dazu kommen, dass ein durch die Recheneinheit 420 ermittelter Betriebsparameter 122 jetzt jedoch zu niedrig liegt. Dies wird in Operation 512 kontrolliert.

Zur Durchführung von Operation 512 ist die Recheneinheit 420 der Steuereinheit 200 ausgebildet, nach ermitteln des Betriebsparameters 122 festzustellen, ob der Betriebsparameter 122 außerhalb des durch die Speichereinheit 410 bereitgestellten Betriebsparametersollbereichs 416 liegt. Die Betriebsparametersollbereich 416 kann beispielsweise durch Angabe einer Untergrenze, wie einer Untergrenze für die Drehzahl, gegeben sein.

Liegt der Betriebsparameter 122 nicht außerhalb des Betriebsparametersollbereichs 416, kann die Steuerung mit unverändertem Druckdifferenzsollwert 112 fortgesetzt werden, wie in Fig. 5 mit Rückkehr zur Operation 506 angedeutet ist.

Falls der Betriebsparameter 122 jedoch außerhalb des Betriebsparametersollbereichs 416 liegt, ist die Recheneinheit 120 ausgebildet, den Druckdifferenzsollwert 112 in einer Operation 514 anzupassen. Hierzu ist die Recheneinheit 514 ausgebildet, den Druckdifferenzsollwert durch Operation 514 so zu reduzieren, dass die Förderrate der Blutpumpe erhöht wird. Im Anschluss erfolgt über Operation 502 die Fortführung des Regelungsschemas mit dem angepassten Druckdifferenzsollwert 112.

Ist die rechte Herzhälfte des Patienten beeinträchtigt, kann es dazu kommen, dass die Förderleistung des Betriebsparameters stetig verringert wird und es dennoch zu einem Ansaugereignis kommt. Dies deutet darauf hin, dass die Regelung der Herzpumpe im Differenzwertbetriebsmodus ungeeignet ist.

Aus diesem Grund kann die Recheneinheit 420 ausgebildet sein, zu überwachen, ob ein Ansaugereignis vorliegt und gleichzeitig der Betriebsparameter 122 außerhalb eines Betriebsparametersollbereichs liegt, oder ob das Ansaugereignis und der Betriebsparameter 122 außerhalb der Betriebsparametersollbereichs alternierend auftreten. Dies ist mit Schritt 516 im Regelungsschema der Fig. 5 angedeutet. Ist diese Bedingung erfüllt, kann die Recheneinheit 120 ebenfalls mittels Operation 590 in den anderen, vom Differenzwertbetriebsmodus verschiedenen Betriebsmodus wechseln. Das Überprüfen, ob Ansaugereignis und Betriebsparameter außerhalb des Betriebsparametersollbereichs alternierend auftreten, kann beispielsweise anhand eines vorgegebenen Zeitintervalls und einer vorgegebenen maximalen Anzahl von Wechseln zwischen Ansaugereignis und Betriebsparameter außerhalb des Betriebsparametersollbereichs über das vorgegebene Zeitintervall erfolgen.

Im in Fig. 5 gezeigten Regelungsschema ist die Recheneinheit 420 ausgebildet, vor dem Wechsel des Betriebsmodus eine Operation 518 ein Ansaugloslassverfahren auszuführen und in einer Operation 520 zu ermitteln, ob das Ansaugloslassverfahren erfolgreich war. Ferner ist die Recheneinheit 420 ausgebildet, nur dann wenn das Ansaugloslassverfahren nicht erfolgreich war, in den anderen Betriebsmodus zu wechseln. War das Ansaugloslassverfahren jedoch erfolgreich, ist die Recheneinheit 420 ausgebildet, mit Operation 502 die Steuerung im Differenzwertbetriebsmodus fortzusetzen. Hierdurch kann verhindert werden, dass der Differenzwertbetriebsmodus zu schnell verlassen wird, wenn die rechte Herzhälfte des Patienten ausreichend funktionsfähig ist und nur ein behebbares Ansaugereignis vorliegt. Eine Möglichkeit dafür, wie ein solches Ansaugloslassverfahren realisiert werden kann, ist beispielsweise in US 2016/058929 A1 offenbart.

Wie auch bei der Steuereinheit 100 kann die Recheneinheit 420 der Steuereinheit 400 durch eine anwendungsspezifische integrierte Schaltung (engl. ASIC) ausgebildet sein, bei der die Steuerstrategie zumindest teilweise durch die Hardware vorgegeben ist. Alternativ kann die Recheneinheit 420 auch mittels eines Mikrokontrollers oder eines Prozessors realisiert werden, bei dem die Steuerungsstrategie durch ein Computerprogramm vorgegeben ist. Bei der Speichereinheit 410 kann es sich um einen flüchtigen Speicher, wie beispielsweise einem Arbeitsspeicher, oder einen nicht flüchtigen Speicher, wie beispielsweise einer Festplatte, handeln. Die Speichereinheit 410 kann auch Teil eines Prozessors, Mikrokontrollers oder ASICs sein, mittels dem die Recheneinheit 420 realisiert ist. Wie die Steuereinheit 100 kann auch die Steuereinheit 400 sowohl als implantierbare Steuereinheit ausgebildet sein als auch um außerhalb des Körpers des Patienten getragen zu werden.

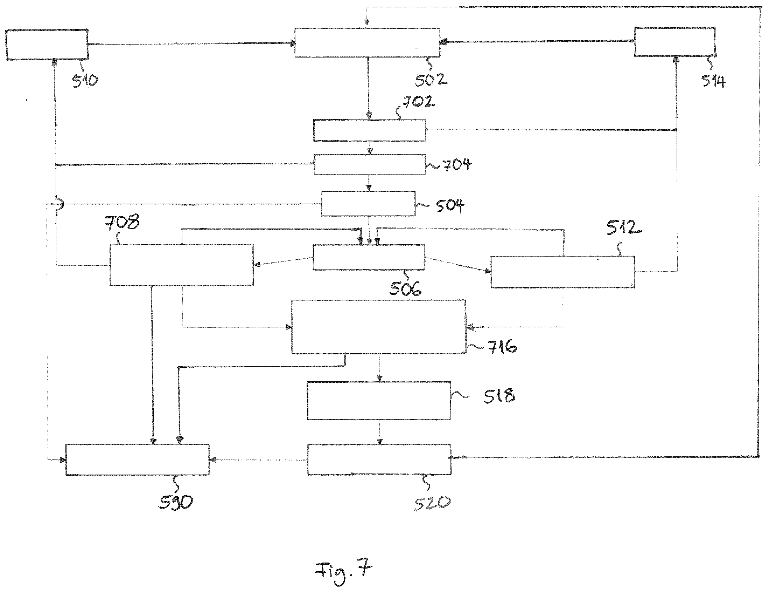

Bei der in Fig. 4 gezeigten Steuereinheit 400 erfolgt die Regelung der Herzpumpe in ersten Linie auf Basis des Druckdifferenzwertes, der sich relativ einfach durch einen am Septum angeordneten Druckdifferenzwertsensor bestimmen lässt. Liegen neben dem Druckdifferenzwert auch Informationen zum Rechtsherzfüllungsdruck vor, können diese zusätzlichen Informationen jedoch genutzt werden, um die Herzpumpe noch genauer steuern. Ein Beispiel für eine Steuereinheit, die auch einen Rechtsherzfüllungsdruck bei der Steuerung der Herzpumpe berücksichtigt, wird im Folgenden anhand der Fign. 6 und 7 beschrieben.

Fig. 6 zeigt eine Schemazeichnung einer Steuereinheit 600, die bei der Steuerung der Herzpumpe den Rechtsherzfüllungsdruck berücksichtigt.

Die Steuereinheit 600 ist weitgehend identisch zur Steuereinheit 400 der Fig. 4. Im Gegensatz zu der Steuereinheit 400 umfasst die Steuereinheit 600 eine Drucksignalschnittstelle 602 die ausgebildet ist, ein erstes Drucksignal und ein zweites Drucksignal zu empfangen, wobei der erste Drucksignal 601R einen Füllungsdruck in der rechten Herzhälfte angibt und das zweite Drucksignale 601L einen Füllungsdruck in der linken Herzhälfte angibt.

Ferner umfasst die Steuereinheit 600 eine Differenzdruckermittlungseinheit 604, die ausgebildet ist, den Druckwert 603R und den Druckwert 603L zu empfangen und in Abhängigkeit dieser Werte den Druckdifferenzwert 104 zu ermitteln.

Weiterhin ist Speichereinheit 610 ausgebildet, neben der Bereitstellung des Druckdifferenzsollwerts 112, des Druckdifferenzsollwertbereichs 414 und des Betriebsparametersollbereichs 416 auch einen ersten Grenzwert 618 und einen zweiten Grenzwert 619 bereitzustellen. Ferner ist die Speichereinheit 610 ausgebildet, einen unteren Rechtsherzfüllungsdruckgrenzwert 617L und einen oberen Rechtsherzfüllungsdruckgrenzwert 617U bereitzustellen.

Zudem umfasst die Steuereinheit 600 eine Recheneinheit 620, die ausgebildet ist, den Druckdifferenzwert 104 und den Druckwert 603R zu empfangen. Wie die Steuerung der Herzpumpe in Abhängigkeit dieser beiden Druckwerte, des ersten und zweiten Grenzwertes 618, 619 sowie des oberen und unteren Rechtsherzfüllungsdruckgrenzwertes 617U, 617L durch die Recheneinheit 620 erfolgt, wird im Folgenden mit Bezugnahme auf Fig. 6 beschrieben.

Fig. 7 zeigt ein Regelungsschema zur Illustration von Operationen, die zur Regelung der Herzpumpe durch die Recheneinheit 620 der in Fig. 6 dargestellten Steuereinheit 600 durchgeführt werden.

Das in Fig. 7 dargestellte Regelungsschema ist weitgehend identisch zu dem in Fig. 5 gezeigte Regelungsschema, weswegen im Folgenden nur auf die Unterschiede eingegangen wird.

Wie in Fig. 7 dargestellt, ist die Recheneinheit 620 ausgebildet, nach dem Ausführen der Operation 502, d.h. dem Abrufen des Druckdifferenzsollwertes aus der Speichereinheit 110, in Operation 702 zu überprüfen, ob der Druckwert 603R größer ist als der durch die Speichereinheit 610 bereitgestellte obere Rechtsherzfüllungsdruckgrenzwert 617U. Ferner ist die Recheneinheit 620 ausgebildet, wenn die Bedingung erfüllt ist, den Druckdifferenzsollwert durch Ausführen der Operation 510 anzupassen. Ein hoher Rechtsherzfüllungsdruck ist ein Indikator dafür, dass die Herzpumpe stärker pumpen muss, um die von der rechten Herzhälfte bereitgestellte Blutmenge in den Körperkreislauf zu transportieren. Durch das Anpassen des Druckdifferenzsollwertes mittels Operation 514 kann die Förderrate der Herzpumpe erhöht werden. Der obere Rechtsherzfüllungsdruckgrenzwert 617U kann beispielsweise bei 20mmHg liegen.

Weiterhin ist die Recheneinheit 620 ausgebildet, zu überprüfen, ob der Druckwert 603R den von der Speichereinheit 610 bereitgestellten unteren Rechtsherzfüllungsdruckgrenzwert 617L unterschreitet. Ferner ist die Recheneinheit 620 ausgebildet, wenn diese Bedingung erfüllt ist, den Druckdifferenzsollwert durch Ausführen der Operation 514 anzupassen. Ein zu niedriger Rechtsherzfüllungsdruck kann zu einem Ansaugereignis führen. Durch die Anpassung des Druckdifferenzsollwertes mittels Operation 510 kann dieses Risiko verringert werden. Der untere Rechtsherzfüllungsdruckgrenzwert 617L kann beispielsweise bei 5mmHg liegen.

Sind die Bedingungen der Operationen 502 und 504 nicht erfüllt und liegt der Druckdifferenzsollwert innerhalb des Druckdifferenzsollbereichs, erfolgt die Steuerung der Blutpumpe anhand des unveränderten Druckdifferenzwertes in Operation 506.

Weiterhin unterscheidet sich das Regelungsschema der Fig. 7 vom Regelungsschema der Fig. 5 durch Operation 708. Zur Durchführung der Operation 708 ist die Recheneinheit 620 ausgebildet, wie in Operation 508 zu überprüfen, ob ein Ansaugereignis vorliegt. Zudem ist die Recheneinheit 620 ausgebildet in Operation 708 zu überprüfen, ob der Druckwert 603R kleiner ist als der durch die Speichereinheit 610 bereitgestellte erste Grenzwert 618. Die Recheneinheit 720 ist ausgebildet, nur wenn beide Bedingungen erfüllt sind, eine Anpassung des Druckdifferenzsollwertes durch Operation 510 auszuführen. Weiterhin ist die Recheneinheit 620 ausgebildet, wenn ein Ansaugereignis festgestellt wurde, aber der Druckwert 603R oberhalb des ersten Grenzwertes 618 liegt, mittels Operation 590 in den Konstantwertbetriebsmodus zu wechseln. Ist nämlich der Rechtsherzfüllungsdruck zu niedrig, könnte eine weitere Reduzierung des Druckdifferenzsollwertes 112 zu einer zu geringen Pumpenleistung führen. Um dies zu verhindern, wird in den Konstantwertbetriebsmodus gewechselt. Der erste Grenzwert 618 kann beispielsweise 18mmHg betragen.

Schließlich unterscheidet sich das Regelungsschema der Steuereinheit 600 von dem Regelungsschema der Steuereinheit 400 auch in Operation 716. Zur Durchführung von Operation 716 ist die Recheneinheit 620 wie auch zur Durchführung der Operation 516 ausgebildet, zu überwachen, ob ein Ansaugereignis vorliegt und gleichzeitig der Betriebsparameter 122 außerhalb eines Betriebsparametersollbereichs liegt, oder ob das Ansaugereignis und der Betriebsparameter 122 außerhalb der Betriebsparametersollbereichs alternierend auftreten. Zudem ist die Recheneinheit 620 zur Durchführung der Operation 716 auch ausgebildet, zu überprüfen, ob der Druckwert 603R über dem durch die Speichereinheit 610 bereitgestellten zweiten Grenzwert 619 liegt. Nur wenn beide Bedingungen erfüllt sind, wird das Ansaugloslassverfahren mittels Operation 518 ausgeführt. Liegt der Druck im rechten Vorhof nicht über dem zweiten Grenzwert, wird über Operation 590 sofort der Betriebsmodus gewechselt. Der zweite Grenzwert 619 kann beispielsweise bei 20mmHg liegen. Durch die Überprüfung, ob der Druckwert 603R über dem zweiten Grenzwert 619 liegt, kann ermittelt werden, ob ein Ansaugereignis stattgefunden hat, weil die Herzpumpe zu viel gepumpt hat oder weil die Herzpumpe falsch platziert ist. Bei ersterem Ereignis ist zu erwarten, dass der Druckwert 603R geringer als der zweite Grenzwert 619 ist. Die Durchführung eines Ansaugloslassverfahrens ist hier sinnvoll. Liegt der Druckwert 603R jedoch oberhalb des zweiten Grenzwertes, ist es wahrscheinlich, dass die Pumpe falsch platziert ist. In diesem Fall würde auch ein Ansaugloslassverfahren das Problem nicht beheben, weshalb direkt in den anderen Betriebsmodus gewechselt wird.

Wie auch bei der Steuereinheit 100 und 400 kann die Recheneinheit 620 der Steuereinheit 600 durch eine anwendungsspezifische integrierte Schaltung (engl. ASIC) ausgebildet sein, bei der die Steuerstrategie zumindest teilweise durch die Hardware vorgegeben ist. Alternativ kann die Recheneinheit 620 auch mittels eines Mikrokontrollers oder eines Prozessors realisiert werden, bei dem die Steuerungsstrategie durch ein Computerprogramm vorgegeben ist. Bei der Speichereinheit 610 kann es sich um einen flüchtigen Speicher, wie beispielsweise einem Arbeitsspeicher, oder einen nicht flüchtigen Speicher, wie beispielsweise einer Festplatte, handeln. Die Speichereinheit 610 kann auch Teil eines Prozessors, Mikrokontrollers oder ASICs sein, mittels dem die Recheneinheit 620 realisiert ist. Wie die Steuereinheit 100 und 400 kann auch die Steuereinheit 600 sowohl als implantierbare Steuereinheit ausgebildet sein als auch um außerhalb des Körpers des Patienten getragen zu werden.

Abschließend soll noch einmal auf die Fig. 6 zurückgekommen werden. In Fig. 6 ist zu sehen, dass die Steuereinheit 600 weiterhin eine Eingabeschnittstelle 692 umfasst, die ausgebildet ist, einen Eingabewert 693 zu empfangen. Ferner ist die Recheneinheit 620 ausgebildet, in einem Initialisierungsmodus einen Startwert 694 für den Druckdifferenzsollwert zu ermitteln und in der Speichereinheit 610 zu hinterlegen. In dem in Fig. 6 gezeigten Ausführungsbeispiel handelt es sich bei dem Eingabewert 693 um einen Betriebsparameterwert wie bspw. die Drehzahl zum Einstellen einer Förderrate der Herzpumpe. In diesem Ausführungsbeispiel ist die Recheneinheit 620 zusätzlich ausgebildet, im Initialisierungsmodus das Steuersignal 122 entsprechend des empfangenen Betriebsparameterwertes 693 bereitzustellen und die sich aufgrund des Betriebsparameterwertes einstellenden Füllungsdruckdifferenz zu ermitteln und als Startwert 694 zu verwenden. Alternativ hierzu kann es sich in anderen Ausführungsformen bei dem Eingabewert 693 direkt um den zu hinterlegenden Startwert für den Druckdifferenzsollwert handeln. Bei dem in Fig. 6 gezeigten Beispiel handelt es sich bei der Eingabeschnittstelle um eine Datenübertragungsschnittstelle zum Anschluss einer externen Eingabeeinheit wie einer Tastatur. Alternativ kann es sich bei der Eingabeschnittstelle, jedoch auch um eine interne Eingabeschnittstelle handeln, die in Verbindung mit einer in die Steuereinheit integrierten Eingabeeinheit steht, so dass der Druckdifferenzsollwert direkt in die Steuereinheit eingegeben werden kann. Auch ist es in anderen Ausführungsbeispielen auch möglich, dass alle anderen Grenzwerte und im Regelschema verwendeten Wertebereiche über die Eingabeschnittstelle bereitgestellt werden können.

Ferner umfasst die Steuereinheit 600 eine Ausgabeschnittstelle 691. Zudem ist die Speichereinheit 610 ausgebildet, den in der Speichereinheit 610 hinterlegten Druckdifferenzsollwert an der Ausgabeschnittstelle 691 bereitzustellen.

Zusammenfassend beschreibt diese Offenbarung eine Steuereinheit (100) zur Steuerung einer Herzpumpe zur mechanischen Unterstützung eines Herzens Hierzu umfasst die Steuereinheit (100) eine Drucksignalschnittstelle (102), die ausgebildet ist, ein oder mehrere Drucksignale, die einen Druckdifferenzwert (104) entsprechend eines Füllungsdruckunterschieds zwischen einer rechten Herzhälfte und einer linken Herzhälfte des Herzens angeben, oder anhand derer dieser Druckdifferenzwert ermittelbar ist, zu empfangen. Ferner umfasst die Steuereinheit (100) eine Speichereinheit (110), die ausgebildet ist, einen Druckdifferenzsollwert (112) bereitzustellen. Weiterhin umfasst die Steuereinheit (100) eine Recheneinheit (120), die ausgebildet ist, den Druckdifferenzwert (104) und den Druckdifferenzsollwert (112) zu empfangen und in einem Differenzwertbetriebsmodus einen Betriebsparameter (122) der Herzpumpe in Abhängigkeit des Druckdifferenzwertes (104) und des Druckdifferenzsollwertes (112) zu ermitteln. Schließlich umfasst die Steuereinheit eine Steuersignalschnittstelle (132), die ausgebildet ist, ein Steuersignal, das den ermittelten Betriebsparameter (122) angibt, bereitzustellen.

## Patentansprüche

1. Steuereinheit (100, 400, 600) zur Steuerung einer Herzpumpe zur mechanischen Unterstützung eines Herzens, umfassend:
eine Drucksignalschnittstelle (102), die ausgebildet ist, ein oder mehrere Drucksignale (101), die einen Druckdifferenzwert (104) entsprechend eines Füllungsdruckunterschieds zwischen einer rechten Herzhälfte und einer linken Herzhälfte des Herzens angeben, oder anhand derer dieser Druckdifferenzwert ermittelbar ist, zu empfangen,
eine Speichereinheit (110), die ausgebildet ist, einen Druckdifferenzsollwert (112) bereitzustellen,
eine Recheneinheit (120), die ausgebildet ist, den Druckdifferenzwert (104) und den Druckdifferenzsollwert (112) zu empfangen und in einem Differenzwertbetriebsmodus einen Betriebsparameter (122) der Herzpumpe in Abhängigkeit des Druckdifferenzwertes (104) und des Druckdifferenzsollwertes (112) zu ermitteln, und
eine Steuersignalschnittstelle (132), die ausgebildet ist, ein Steuersignal (133), das den ermittelten Betriebsparameter (122) angibt, bereitzustellen.

2. Steuereinheit (100) nach Anspruch 1, wobei der Betriebsparameter (122) eine Förderrate der Herzpumpe einstellt und/oder beeinflusst.

3. Steuereinheit (100) nach Anspruch 1 oder 2, wobei die Recheneinheit ausgebildet ist,
den Druckdifferenzwert (104) mit dem Druckdifferenzsollwert (112) zu vergleichen und
den Betriebsparameter (122) in Abhängigkeit eines Ergebnisses des Vergleichs zu bestimmen.

4. Steuereinheit (600) nach einem der vorhergehenden Ansprüche, wobei
die ein oder mehreren Drucksignale (601R,L) einen Rechtsherzfüllungsdruck angeben oder anhand derer dieser ermittelbar ist,
die Speichereinheit (110) ausgebildet ist, wenigstens einen Rechtsherzfüllungsdruckgrenzwert (617L,U) bereitzustellen, und
die Recheneinheit (620) ausgebildet ist, den Rechtsherzfüllungsdruck mit dem wenigstens einen Rechtsherzfüllungsdruckgrenzwert (617L,U) zu vergleichen und in Abhängigkeit von einem Ergebnis des Vergleichs, den Druckdifferenzsollwert (112) in der Speichereinheit (610) anzupassen.

5. Steuereinheit (600) nach Anspruch 4, wobei
der wenigstens eine Rechtsherzfüllungsdruckgrenzwert einen unterer Rechtsherzfüllungsdruckgrenzwert (617L) umfasst und die Recheneinheit (620) ausgebildet ist, wenn der Füllungsdruck in der rechten Herzhälfte kleiner ist als der untere Rechtsherzfüllungsdruckgrenzwert (617L), den Druckdifferenzsollwert (112) anzupassen, und/oder
der wenigstens eine Rechtsherzfüllungsdruckgrenzwert einen oberen Rechtsherzfüllungsdruckgrenzwert (617U) umfasst und die Recheneinheit (620) ausgebildet ist, wenn der Füllungsdruck in der rechten Herzhälfte größer ist als der obere Rechtsherzfüllungsdruckgrenzwert (617U), den Druckdifferenzsollwert (112) anzupassen.

6. Steuereinheit (400, 600) einem der vorhergehenden Ansprüche, wobei die Recheneinheit (420, 620) ausgebildet ist,
nach dem Bereitstellen des Steuersignals (133) festzustellen, ob ein Ansaugereignis vorliegt, und
falls das Ansaugergebnis vorliegt, den Druckdifferenzsollwert (112) in der Speichereinheit (410, 610) anzupassen.

7. Steuereinheit (400, 600) nach einem der vorhergehenden Ansprüche, wobei die Recheneinheit (420, 620) ausgebildet ist,
nach ermitteln des Betriebsparameters (122) festzustellen, ob der Betriebsparameter (122) außerhalb eines vorgegebenen Betriebsparametersollbereichs (416) liegt, und
falls der Betriebsparameter (122) außerhalb des Betriebsparametersollbereichs (416) liegt, den Druckdifferenzsollwert (122) in der Speichereinheit (410, 610) anzupassen.

8. Steuereinheit (400, 600) nach einem der vorhergehenden Ansprüche, wobei die Recheneinheit (420, 620) ausgebildet ist,
zu überwachen, ob ein Ansaugereignis vorliegt und gleichzeitig der Betriebsparameter (122) außerhalb eines Betriebsparametersollbereichs (416) liegt, oder ob das Ansaugereignis und der Betriebsparameter (122) außerhalb der Betriebsparametersollbereichs (416) alternierend auftreten, und
in Abhängigkeit von einem Ergebnis des Überwachens, in einen anderen, vom Differenzwertbetriebsmodus verschiedenen Betriebsmodus zu wechseln.

9. Steuereinheit (400, 600) nach Anspruch 8, wobei die Recheneinheit (420, 620) ausgebildet ist, bevor sie in den anderen Betriebsmodus wechselt, ein Ansaugloslassverfahren auszuführen, und nur dann wenn das Ansaugloslassverfahren nicht erfolgreich ist, in den anderen Betriebsmodus zu wechseln.

10. Steuereinheit (400, 600) nach einem der vorhergehenden Ansprüche, wobei die Recheneinheit (420, 620) ausgebildet ist,
vorzugsweise vor dem Bestimmen des Betriebsparameters (122) im Differenzwertbetriebsmodus, zu überprüfen, ob der Druckdifferenzsollwert (112) innerhalb eines vorgegebenen Druckdifferenzsollwertbereichs (414) liegt, und
falls der Druckdifferenzsollwert (112) außerhalb des Druckdifferenzsollbereichs (414) liegt, in einen anderen, vom Differenzwertbetriebsmodus verschiedenen Betriebsmodus zu wechseln.

11. Steuereinheit (400, 600) nach einem der Ansprüche 8 bis 10, wobei der andere Betriebsmodus ein Konstantwertbetriebsmodus ist und die Recheneinheit (420, 620) ausgebildet ist, in dem Konstantwertbetriebsmodus den Betriebsparameter (122) auf einen vordefinierten konstanten Betriebsparameterwert zu setzen.

12. Herzpumpenanordnung (200) umfassend:
die Steuereinheit (100) nach einem der vorhergehenden Ansprüche, und ferner
ein oder mehrere Drucksensoren (210, 212A,B), die ausgebildet sind, die ein oder mehreren Drucksignale (101) an der Drucksignalschnittstelle (102) der Steuereinheit (100) bereitzustellen, und/oder
die Herzpumpe (220), die ausgebildet ist, an der Steuersignalschnittstelle (132) bereitgestellte Steuersignale (133) zu empfangen.

13. Verfahren (300) zum Betreiben einer Herzpumpe zur mechanischen Unterstützung eines Herzens, umfassend die folgenden Schritte:
Empfangen (302) eines oder mehrerer Drucksignale, die einen Druckdifferenzwert entsprechend eines Füllungsdruckunterschieds zwischen einer rechten Herzhälfte und einer linken Herzhälfte des Herzens angeben oder anhand derer dieser Druckdifferenzwert ermittelbar ist,
Bereitstellen (304) eines Druckdifferenzsollwertes,
in einem Differenzwertbetriebsmodus Ermitteln (306) eines Betriebsparameters der Herzpumpe in Abhängigkeit des Druckdifferenzwertes und des Druckdifferenzsollwertes, und
Bereitstellen (308) eines Steuersignals, das den ermittelten Betriebsparameter angibt.
